# EUROPÄISCHE PATENTSCHRIFT

(11) **EP 1 744 681 B1**
(45) Veröffentlichungstag und Bekanntmachung des Hinweises auf die Patenterteilung: **22.06.2011**
(21) Anmeldenummer: 05753713.6
(22) Anmeldetag: 04.05.2005
(51) Int. Cl.: A61B 17/064, A61F 2/08, A61B 17/068

(54) **BLINDNIET ZUR ADAPTION VON BIOLOGISCHEM GEWEBE**
BLIND RIVET FOR ADAPTING BIOLOGICAL TISSUE
RIVET AVEUGLE POUR L'ADAPTATION DE TISSU BIOLOGIQUE

(30) Priorität: 07.05.2004 DE 102004022590
(43) Veröffentlichungstag der Anmeldung: 24.01.2007
(73) Patentinhaber: Technische Universität München, 80333 München (DE)
(72) Erfinder: FEUSSNER, Hubertus, 81671 München (DE); HEINZL, Joachim, 81549 München (DE); HAUSMANN, Ulrich, 73434 Aalen (DE); PASPA, Robert, 85551 Kirchheim (DE)
(74) Vertreter: Czybulka, Uwe
(86) Internationale Anmeldenummer: PCT/DE2005/000846
(87) Internationale Veröffentlichungsnummer: WO 2005/107608

(56) Entgegenhaltungen:
- US-A1- 2001 037 130
- US-A1- 2004 044 364

## Beschreibung

Die Erfindung betrifft einen selbststechenden Blindniet zur Adaption von biologischem Gewebe, um Gewebeschichten mit einstellbarer Anpresskraft zu verbinden.

### Stand der Technik

Blindnieten werden üblicherweise zum Verbinden von Metallblechen - vorwiegend im Flugzeugbau - verwendet, können jedoch auch für andere Materialien, wie Kunststoff, Holz oder Leder, eingesetzt werden. Ihr großer Vorteil gegenüber anderen Nietformen ist, dass die Fügeteile nur von einer Seite zugänglich sein müssen, da keine Matrize zum Schließen des Niets erforderlich ist. Für die meisten Blindnieten müssen zuvor Löcher gebohrt werden, die zum Setzen des Niets übereinander liegen. Es gibt jedoch auch Blindnieten, wie z.B. in der EP 0 705 389 B1, die selbstdurchbohrend sind - beim Setzen also selbständig die Fügeteile durchstechen.

Auch in der Chirurgie gibt es erste Ansätze, Blindnieten einzusetzen. Deren Anwendungen beschränken sich aber hauptsächlich auf die Fixierung von Implantaten oder Gewebe an Knochen, wie es in US 6,241,732 B1 oder WO 99/62418 zu sehen ist. Erwähnenswert ist hier auch ein Kornealniet (US 5,258,011), durch den das Schließen von Schnitten in der Hornhaut des Auges möglich ist. Dieser Niet ist allerdings nicht als Blindniet ausgeführt, sondern muss von beiden Seiten bedient werden, was die Anwendung in der Endoskopie erschwert und ein weiteres Instrument erfordern würde.

Zum Adaptieren von Gewebe werden in der Medizin hauptsächlich Nahttechniken eingesetzt. So wurde versucht, Nähvorrichtungen für die minimalinvasive Chirurgie zu entwickeln, um bei Operationen im gastrointestinalen Trakt Gewebsdiskontinuitäten zu verschließen. Da hier die Nadel nicht mehr mit den Fingern geführt werden kann, muss dies von Greifern übernommen werden. Da aber diese Greifer nicht annähernd die Bewegungsfreiheit und Bewegungsmöglichkeiten der Hand erreichen, ist dieses Nähen sehr schwierig, unsicher und zeitaufwendig. Deshalb wurden verschiedenste Vorrichtungen zum automatisierten Nähen angedacht und zum Teil umgesetzt. Als Beispiel hierzu soll die US 6,071,289 dienen. Der gravierendste Nachteil der Nahttechnik kann jedoch auch hiermit nicht beseitigt werden: das Verknoten des Fadens. Dazu wird entweder ein Knoten außerhalb des Körpers gelegt und durch den Instrumentenkanal hindurchgeschoben, oder der Faden wird mit Greifern mühsam verknotet. Somit konnten solche Vorrichtungen wegen ihrer Komplexität und der daraus folgenden Baugröße noch nicht in den Instrumentenkanälen von flexiblen Endoskopen eingesetzt werden.

Aufgrund der Nachteile der Nähvorrichtungen wurden als Alternative Klammern und Clips entwickelt. Diese bestehen im wesentlichen aus zwei zusammenhängenden Armen, mit denen das Gewebe zusammengehalten wird, wie es in der DE 299 23 545 U1 oder in der DE 102 03 956 A1 zu sehen ist. Da die Arme hierfür nur in einem sehr begrenzten Bereich aufspreizbar sind, ist es nötig, dass die zu fügenden Gewebeteile mit einer weiteren Vorrichtung vor dem Applizieren approximiert werden, wodurch der Einsatz mittels Endoskops unterbunden wird.

Eine Kombination aus Clip und Nähverfahren stellt das Verbindungselement aus der Patentanmeldung US 2003/0125755 dar, wobei am Ende einer Nadel mit Faden ein Clip hängt, der mit einer Zange vom Faden abgelöst wird. Somit wurde das Problem des Knotens bewältigt, allerdings ist wiederum der Einsatz eines weiteren Instrumentes von Nöten, wodurch der Einsatz allein durch den Instrumentenkanal eines Endoskops nicht gewährleistet ist.

Eine weitere Ausführung, die als Blindniet aufgefasst werden kann, stellt die Anmeldung WO 99/60931 dar. Hierbei besteht ein Clip aus einem Träger, einem distalen und proximalen Fixierelement. Für die Funktion ist hier auch noch eine Verbindungshülse zwischen den beiden Fixierelementen vorgesehen. Die Hülse bestimmt den Abstand der Fixierelemente, weshalb das Ausmaß der Gewebeapproximation starr ist. Es ist somit nicht möglich, mit ein und demselben Clip Gewebe unterschiedlicher oder unbekannter Dicke miteinander zu verbinden, was eine große Einschränkung für den Chirurg darstellt. Miteinher geht hiermit auch, dass die Anpresskraft, mit der das Gewebe zusammengehalten wird, nicht einstellbar ist, wodurch der Chirurg kein Gefühl über die Güte der Verbindung gewinnen kann. Obendrein ist auch die Funktionssicherheit dieses Clips bedenklich, da kein Anschlag für das proximale Fixierelement vorgesehen ist, so dass es vorkommen kann, dass der komplette Clip durch das Gewebe hindurchgeschoben werden kann und sozusagen auf der anderen Seite herausfällt. Auch ist es hierbei nicht möglich, die Clips zu magazinieren, also mit einem Instrument mehrere Clips nacheinander zu setzen. Zum Nachladen muss somit das gesamte Instrument ausgetauscht werden oder zumindest aus dem Endoskop herausgenommen werden.

Aus der veröffentlichten Patentanmeldung US2004/0044364A1 ist ein Blindniet für chirurgische Anwendungen bekannt, der zur Verbindung von Gewebeschichten eingesetzt wird. Hierzu sind auf einem Träger zwei Spangen vorgesehen, wobei die distale Spange fest an dem Träger montiert und als Spitze zum Penetrieren des Gewebes ausgebildet ist und die proximale Spange z. B. durch einen Seilzug nach Penetrieren der Gewebeschichten an die jeweilige Gewebeschicht angelegt und in ihrer Endposition fixiert werden kann. Wenn mehrere Nieten gesetzt werden müssen, was bei Operationen nicht ungewöhnlich ist, muss für jeden Niet das Führungsrohr, üblicherweise ein Endoskop, neu platziert und mit einem neuen Niet ausgestattet werden. Diese Arbeiten kosten Zeit und verzögern die Fortschritte bei der Operation.

Der Erfindung liegt die Aufgabe zugrunde, eine einfache Konstruktion für einen Blindniet anzugeben, mit der die zuvor erwähnten Nachteile vermieden werden. Darüber hinaus soll es möglich sein, sowohl eine beliebige Anzahl von Gewebeschichten beliebiger Dicke mit einer vom Chirurgen einstellbaren Anpresskraft zu verbinden und bequem für den Einsatz in einem Instrumentenkanal eines üblichen, flexiblen Endoskops geeignet zu sein und auch die Arbeiten beim Setzen von mehreren Nieten zu vereinfachen.

Diese Aufgaben werden mit dem nach Anspruch 1 formuliertem selbststechenden Blindniet erfüllt.

Bevorzugte Varianten und Ausführungen werden in den abhängigen Ansprüchen aufgeführt.

Der Blindniet weist somit einen rohrförmigen Träger auf, an dessen distalem Ende eine Spange fest angeordnet ist, wobei die Spitze des Trägers als Nadel ausgebildet ist, mit der das Gewebe durchstochen wird. Nach dem Durchstechen des Gewebes legt sich die distale feste Spange an das Gewebe an und verhindert die Möglichkeit, den Niet zurück zu ziehen. Auf der proximalen Seite des Trägers ist eine Halterung vorgesehen, die sich auf der gegenüber liegenden Seite des Gewebes an diesem abstützt und damit die Lage des Nietes fixiert. Diese proximale Halterung ist üblicherweise eine Spange, die ähnlich aufgebaut ist wie die feste distale Spange, wenn es nur auf das Approximieren von Gewebeschichten ankommt.

Die Halterung kann jedoch auch eine andere Vorrichtung sein oder mit dieser kombiniert werden; so ist es möglich, diese Halterung etwa als Medikamentenspender, als Diagnoseeinrichtung mit Telemetrieübertragung etc. auszubilden.

Die Erfindung wird im folgenden ausführlicher unter Bezugnahme auf die beigefügten Zeichnungen beschrieben, die einige, nichteinschränkende Ausführungen zeigen.
- Fig.1: Aufbau des selbststechenden Blindniets;
- Fig.2: Niet im eingeklappten Zustand;
- Fig.3: erster Gewebelappen aufgefädelt und fixiert;
- Fig.4: Schließen des Niets;
- Fig.5: Niet im appliziertem Zustand;
- Fig.6: Ausnehmung mit Betätigungselements;
- Fig.7: Einrasten des Betätigungselements in der Ausnehmung;
- Fig.8: Abkoppeln des Betätigungselements von der Ausnehmung;
- Fig.9: Abwerfen der Spitze;
- Fig.10: Umklappen der Spitze;
- Fig.11: Verdecken der Spitze.

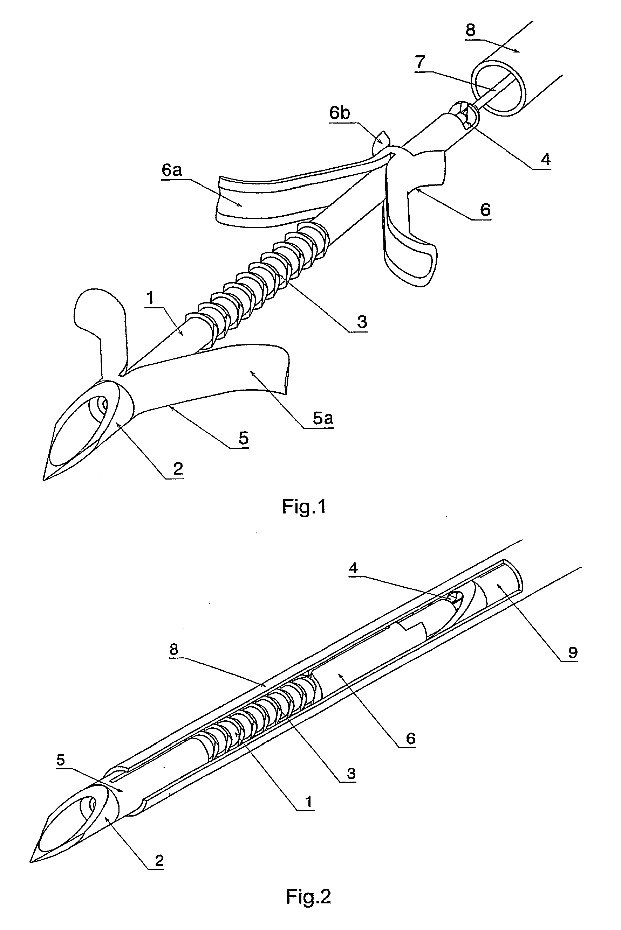

Der Niet, wie ihn Fig.1 in seiner entspannten Form zeigt, besteht im wesentlichen aus drei Teilen. Ein rohrförmiger Träger **1** ist am distalen Ende versehen mit einer Spitze **2,** im Mittelteil mit einer Rastverzahnung **3** und am proximalen Ende mit einem Kupplungselement **4.** Auf dem Träger sind zwei Spangen **5** und **6** angebracht, wobei die distale Spange **5** axial fest gelagert ist und die proximale Spange **6** sich in distaler, axialer Richtung verschieben lässt und in der Gegenrichtung von der Rastverzahnung festgehalten wird. Die distale Spange besteht aus einem zylindrischen Grundkörper, von dem aus sich zwei Schenkel **5a** abspreizen. Die proximale Spange besteht ebenfalls aus einem zylindrischen Grundkörper, von dem aus sich sowohl zwei Schenkel **6a** als auch zwei Flügel **6b** abspreizen. Innerhalb des Trägers befindet sich ein Betätigungselement **7** mit einer Raste am distalen Ende, womit es am Kupplungselement einhaken und den Träger in proximale Richtung ziehen kann. Geführt wird dieses Betätigungselement durch ein Führungsrohr **8.**

Im geschlossenen Zustand, wie ihn Fig.2 mit aufgeschnittenen Führungsrohr **8** darstellt, sind die beiden Spangen **5** und **6** an den Träger **1** durch elastische Verformung angelegt, so dass die Niete im Führungsrohr verschoben werden können. Dabei sind die Niete so ineinander gesteckt, dass das proximale Ende des vorausgehenden Niets in der Spitze des nachfolgenden Niets **9** steckt. Somit kann das Vorschieben der Niete über Schub am Träger des letzten in der Reihe befindlichen Niets erfolgen. Das Zurückziehen der Niete ist ebenfalls möglich, da das Betätigungselement **7** im Kupplungselement **4** des ersten Niets einhakt. Durch das Ineinanderstecken der Niete ergibt sich aufgrund von Spiel ein Gelenk, das zusammen mit der Elastizität des Trägers die Beweglichkeit des Instrumentenkanals des Endoskops erhält. Durch die Geometrie der Spitze und einer Abdeckung hierfür wird vermieden, dass die Spitze das Führungsrohr verletzt, insbesondere wenn dieses gekrümmt ist.

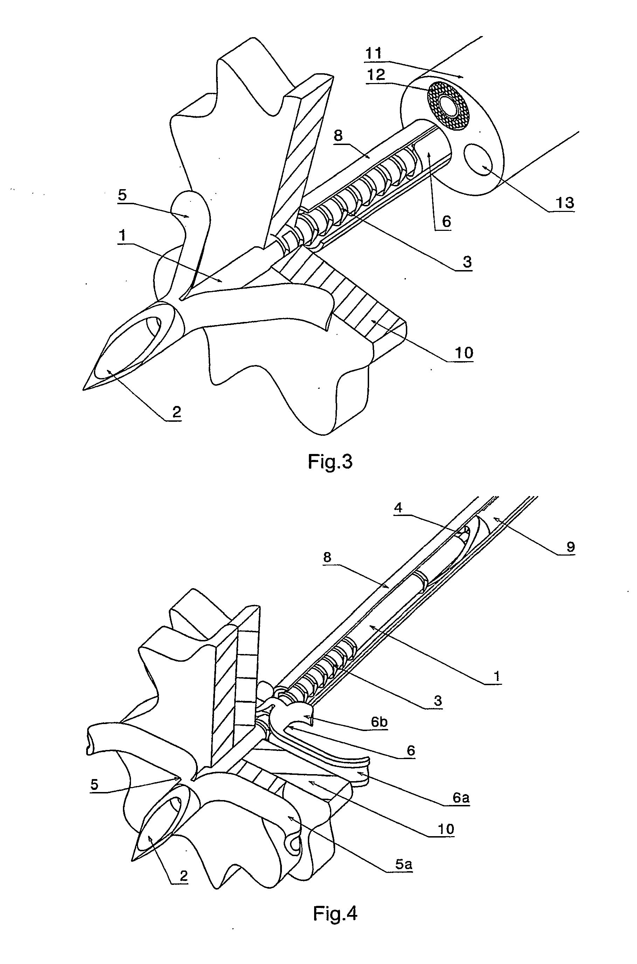

Wird der Niet aus dem Führungsrohr **8** herausgeschoben, so öffnet sich, wie in Fig.3 zu sehen, die distale Spange **5** durch elastische Vorspannung oder durch den Memory-Effekt von Formgedächtnismaterialien. Mit der Spitze **2** werden der Reihe nach Gewebeschichten **10** durchstochen und von der distalen Spange und dem Führungsrohr gesichert. Dadurch ist es möglich, die Gewebeschichten mit der Nietvorrichtung ohne Zusatzinstrumente zu -approximieren.

Es ist auch möglich, zum Setzen des Niets einen Gegenhalter zu verwenden, der z. B. in dem Instrumentenkanal des Endoskopes geführt ist und die zu approximierenden Gewebeschichten umgreift, so dass beim Einstechen der Nadel das Gewebe nicht ausweichen kann. Die Nadel mit dem Blindniet kann im selben Instrumentenkanal innerhalb des Gegenhalters geführt werden; ebenso kann hierfür ein weiterer Instrumentenkanal vorgesehen sein.

Des weiteren ist in Fig.3 ein Endoskop **11,** in dessen Instrumentenkanal sich die Nietvorrichtung befindet, dargestellt. Dieses ist mit einer Optik **12** und einem Spülkanal **13** ausgestattet. Somit befindet sich die Nietvorrichtung im Sichtbereich des Endoskops. Auch kann der Instrumentenkanal des Endoskops die Aufgabe des Führungsrohrs übernehmen.

Das Schließen des Niets, wie in Fig.4 zu sehen, erfolgt dadurch, dass der Niet soweit aus dem Führungsrohr **8** herausgeschoben wird, bis sich zuerst die Schenkel **6a** und dann die Flügel **6b** öffnen. Mit den Flügeln stützt sich die proximale Spange **6** am Führungsrohr ab, wenn der Träger **1** in proximale Richtung gezogen wird. Dies geschieht durch Zug am Betätigungselement **7,** welches im Kupplungselement **4** eingehakt ist. Die proximale Spange wird so über die Rastverzahnung **3** geschoben. Dabei legen sich die Schenkel **5a** und **6a** an das Gewebe **10** an und spreizen sich bei weiterem Andrücken auf, bis sie etwa im 90°Winkel zum Träger stehen. Bei diesem Vorgang erhöht sich die Anpresskraft der Gewebeschichten kontinuierlich. Bei weiterem Anziehen wird die Elastizität des Gewebes ausgenützt, um die Anpresskraft weiter zu erhöhen.

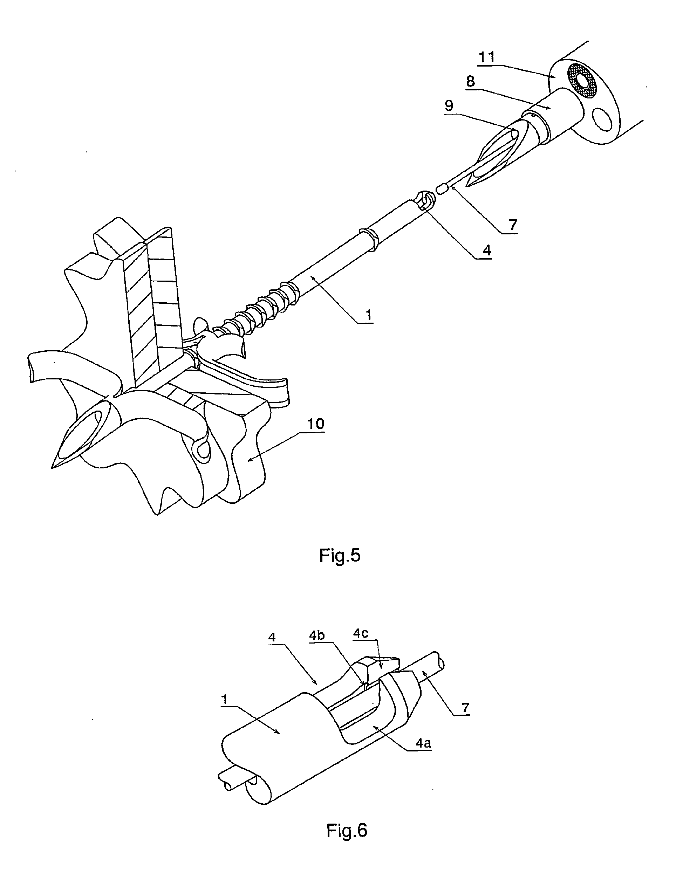

Um den Niet nun von der Vorrichtung abzulösen, wie er in Fig.5 zu sehen ist, wird das Betätigungselement **7** locker gelassen und das Führungsrohr **8** leicht in proximale Richtung gezogen, wodurch sich der nachfolgende Niet **9** vom gerade applizierten ablöst. Somit wird das Kupplungselement **4** freigegeben, und das Betätigungselement kann ausgehängt werden. Der Niet ist nun vollständig appliziert. Dann wird das Betätigungselement soweit in proximale Richtung gezogen, bis es im Kupplungselement des nächsten Niets einhängt. Die Niete werden im Führungsrohr in distale Richtung geschoben, bis sich die distale Spange des nächste Niets öffnet, so dass dieser bereit ist, appliziert zu werden.

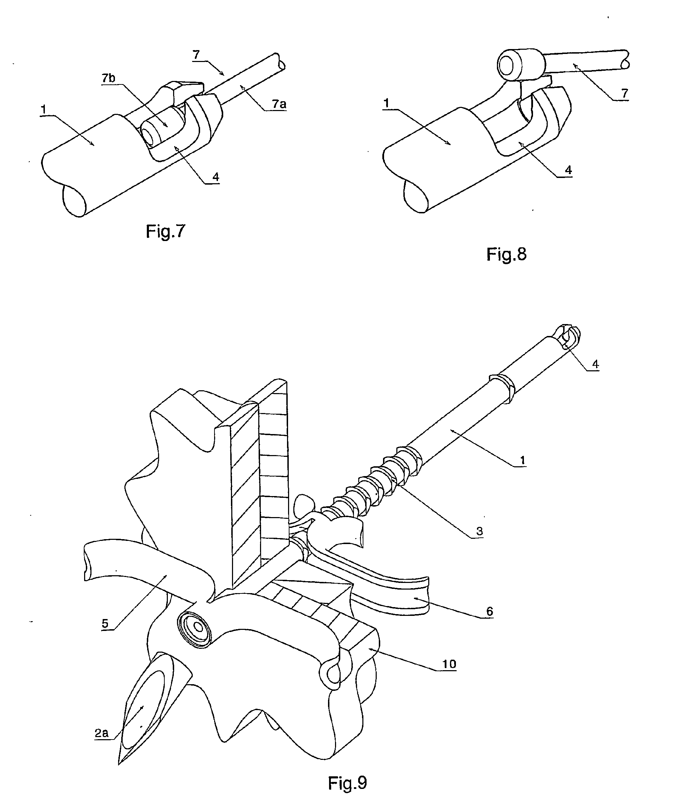

Der Aufbau des Kupplungselements **4** am proximalen Ende des Trägers 1 ist in Fig.6 dargestellt. Es besteht aus einer Ausnehmung **4a,** einem Anschlag **4b** und einer Nut **4c.** Durch das Kupplungselement hindurch verläuft das Betätigungselement **7.** Wie in Fig.7 zu sehen, befindet sich am distalen Ende des Betätigungselements **7,** welches im wesentlichen ein Zugseil **7a** ist, eine Raste **7b.** Diese Raste hakt im Anschlag der Kupplung ein, wenn am Betätigungselement gezogen wird. Wird das Betätigungselement locker gelassen, wie in Fig.8 zu sehen, und zum Träger verkippt, so löst sich die Raste vom Anschlag, womit der Niet vom Betätigungselement und somit von der ganzen Vorrichtung abgelöst wird.

Die Spitze **2** eines applizierten Niets stellt eine Gefahr für umliegendes Gewebe dar und kann zur mechanischen Irritation (Blutung, Perforation) des selbigen führen. Es gibt mehrere Möglichkeiten, dies zu vermeiden.

Die erste Möglichkeit ist, dass die Spitze aus bioresorbierbarem Material hergestellt ist, welches innerhalb weniger Tage oder Stunden vom Körper abgebaut wird; dies ist zum Beispiel der Fall bei verschiedenen Magnesiumlegierungen wie AZ91. Auch Kunststoffe, wie verschiedene Lactide, sind hierfür geeignet; Magnesiumlegierungen bieten jedoch den Vorteil einer zum Einstechen in das Gewebe und zum Durchdringen des Gewebes benötigten Härte.

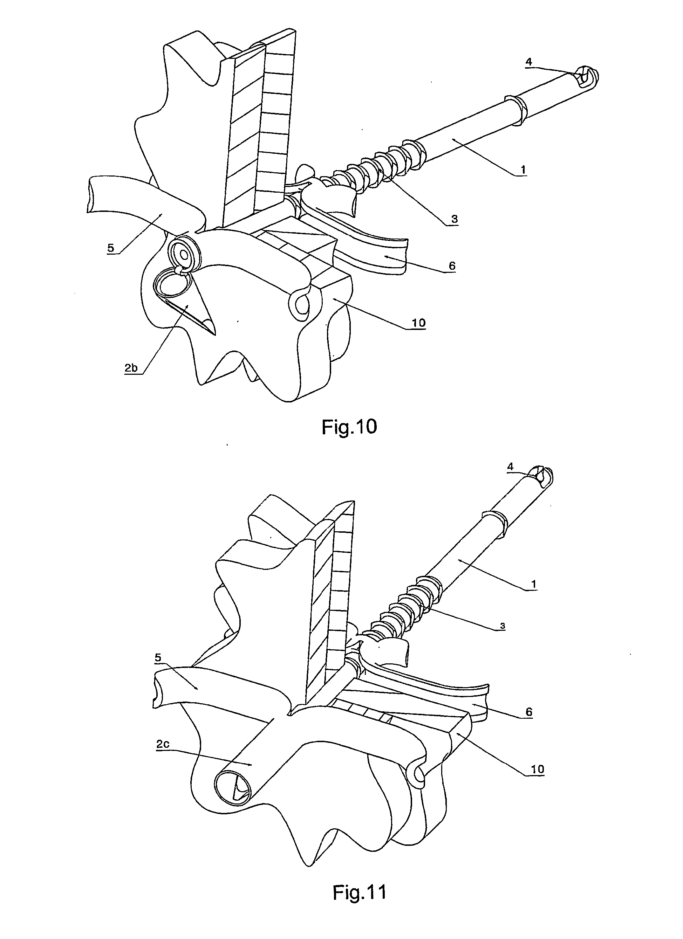

Nach Fig.9 kann die Spitze auch abgeworfen werden. Dies ist realisierbar, indem die Spitze auf dem Träger **1** aufgeschoben ist, und beim Schließen des Niets die distale Spange **5** die Spitze vom Träger herunterschiebt. Die abgeworfene Spitze wird dann, falls der Niet im Gastrointestinaltrakt eingesetzt wird, ausgeschieden werden. Eine weitere Möglichkeit, wie in Fig.10, besteht darin, dass die Spitze umklappt, und somit die Gefahr der Traumatisierung verringert wird. Die letzte Möglichkeit stellt das Verdecken der Spitze dar, wie in Fig.11 zu sehen. Hierbei wird der zylindrische Grundkörper der distalen Spange **5** mit einer Hülse verlängert, die sich beim Schließen des Niets über die Spitze schiebt, diese verdeckt und benachbartes Gewebe auf Distanz hält.

Die verwendeten Materialien sind von großer Bedeutung. Für alle Bestandteile des Niets, also für den Träger **1,** die Spitze **2** und die beiden Spangen **5,6,** müssen biokompatible Werkstoffe eingesetzt werden, um Inflammationen zu vermeiden. Darüber hinaus ist es erwünscht, dass der Niet im Körper verbleiben kann und nach Abschluss des Wundheilungsprozesses abgebaut wird. Aus diesem Grund wird der Träger **1** aus bioresorbierbarem Poly-(D/L)-Lactid hergestellt, was nach mehreren Monaten abgebaut wird. Die beiden Spangen werden aus einem aliphatischen Polyetherbasiertem oder Polycarbonatbasiertem TPU hergestellt. Für den Fall, dass die Spitze sehr schnell resorbiert werden soll, wird, wie bereits erwähnt, die Magnesiumlegierung AZ91 verwendet. Das Betätigungselement **7** wird aus Federstahl hergestellt, um Zugkräfte übertragen zu können. Für das Führungsrohr **8** eignet sich ein reibungsarmer Kunststoff, wie zum Beispiel PTFE.

Wie bereits oben erwähnt, kann anstelle der zweiten Spange eine andere Halterung bzw. ein Gerät vorgesehen werden, wie ein Medikamentenspender etc. Die distale Spange dient in diesem Falle im Wesentlichen dazu, dieses Gerät im Gewebe zu fixieren.

### Bezugszeichenliste

- Träger: **1**
- distales Ende des: **2**
- Trägers Rastverzahnung: **3**
- Kupplungselement: **4**
- Ausnehmung: **4a**
- Anschlag: **4b**
- Nut: **4c**
- distale Spange: **5**
- Schenkel der distalen: **5a**
- Spange proximale Spange: **6**
- Schenkel der proximalen: **6a**
- Spange Flügel der proximalen: **6b**
- Spange Betätigungselement: **7**
- Führungsrohr: **8**
- nachfolgender Niet: **9**
- Gewebe: **10**
- Endoskop: **11**
- Optik: **12**
- Spülkanal: **13**

## Patentansprüche

1. Selbststechender Blindniet zur Adaption von biologischem Gewebe, mit einem Träger **(1),** auf dem eine axial feste distale Spange **(5)** und eine in distaler Richtung axial verschiebliche proximale Spange **(6)** angeordnet sind, wobei der Träger **(1)** am proximalen Ende ein Kupplungselement (4) aufweist, in das zum Verschieben der Spangen relativ zueinander ein Betätigungselement **(7)** einhängbar ist, das aus dem Kupplungselement zum Ablösen des Niets nach dem Setzen aushängbar ist, und wobei das distale Ende des Blindniets als Spitze **(2)** ausgeführt ist, um die Gewebeschichten zu penetrieren, und wobei der Blindniet in einem Führungsrohr **(8)** gehalten werden kann und zum Penetrieren des Gewebes aus diesem herausgeschoben werden kann, **dadurch gekennzeichnet, dass** der Träger **(1)** insgesamt rohrförmig und an seinem distalen Ende als die Spitze **(2)** ausgebildet ist, sodass mehrere Blindniete derart magazinierbar sind, daß das proximale Ende eines vorangehenden Blindniets in die Spitze eines nachfolgenden Blindniets steckbar ist.

2. Blindniet nach Anspruch 1, **dadurch gekennzeichnet, dass** das Betätigungselement **(7)** seilförmig ist und an seinem distalen Ende ein in das Kupplungselement **(4)** eingreifendes Rastelement **(7b)** aufweist.

3. Blindniet nach Anspruch 1 oder 2, **dadurch gekennzeichnet, dass** mehrere Nieten hintereinander anordenbar und in dem Führungsrohr **(8)** in dessen Längsrichtung magaziniert sind, wobei sich das proximale Ende eines vorausgehenden Niets auf dem distalen Ende eines nochfolgenden Niets abstützt, und dass sich das Betätigungselement **(7)** durch die rohrförmigen Träger **(1, 9)** aller magazinierten Nieten erstreckt und nach Aushängen aus dem Kupplungselement **(4)** des vordersten gesetzten Niets in das Kupplungselement **(4)** des nachfolgenden Niets einhängbar ist.

4. Blindniet nach einem der vorhergehenden Ansprüche, **dadurch gekennzeichnet, dass** der Blindniet in das Führungsrohr **(8)** in den Instrumentenkanal eines Endoskops eingeführt ist.

5. Blindniet nach Anspruch 4, **dadurch gekennzeichnet, dass** das Führungsrohr **(8)** der Instrumentenkanal des Endoskops ist.

6. Blindniet nach Anspruch 1, **dadurch gekennzeichnet, dass** sich auf dem rohrförmigen Träger **(1)** eine Rastverzahnung **(3)** befindet, die eine axiale Relativbewegung der proximalen Spange **(6)** nur in distaler Richtung zulässt.

7. Blindniet nach einem der vorhergehenden Ansprüche, **dadurch gekennzeichnet, dass** zum Schließen des Blindniets mit dem Betätigungselement **(7)** am Kupplungselement **4** gezogen wird, wodurch die proximale Spange **(6)in** distaler Richtung verschoben wird.

8. Blindniet nach einem der vorhergehenden Ansprüche, **dadurch gekennzeichnet, dass** die Spitze (2) an dem distalen Ende des Blindniets abgedeckt ist.

9. Blindniet nach einem der vorhergehenden Ansprüche, **dadurch gekennzeichnet, dass** der Träger (1) Verjüngungen und Festkörpergelenke aufweist, die so ausgebildet ist, dass die Bewegungsfreiheit des Führungsrohrs nicht eingeschränkt ist.

10. Blindniet nach einem der vorhergehenden Ansprüche, **dadurch gekennzeichnet, dass** für die Komponenten des Niets biokompatible Materialien eingesetzt sind, wobei zumindest der Träger **(1)** bioresorbierbar ist, sodass der Niet die Schließkraft so lange aufrecht hält, bis das Gewebe selbst tragfähig ist.

11. Blindniet nach Anspruch 10, **dadurch gekennzeichnet, dass** die Spitze **(2)** am distalen Ende des Blindniets, um die Traumatisierung des Gewebes oder der umliegenden Organe zu minimieren, wesentlich schneller als der restliche Niet resorbiert wird.

12. Blindniet nach Anspruch 10, **dadurch gekennzeichnet, dass** die Spitze (2) an dem distalen Ende des Blindniets, um die Traumatisierung des Gewebes oder der umliegenden Organe zu minimieren, nach Setzen des Blindniets abgeworfen wird.

13. Blindniet nach Anspruch 2, **dadurch gekennzeichnet, dass** die Spitze **(2)** an dem distalen Ende des Blindniets, um die Traumatisierung des Gewebes oder der umliegenden Organe zu minimieren, nach Setzen des Blindniets umgeklappt wird.

14. Blindniet nach einem der vorhergehenden Ansprüche, **dadurch gekennzeichnet, dass** die proximale Spange **(6)** mit einem medizinischem Gerät, z. B. einem Medikamentenspender kombiniert ist.

15. Blindniet nach einem der vorhergehenden Ansprüche, **dadurch gekennzeichnet, dass** zum Befestigen des Blindniets ein Gegenhalter vorgesehen ist, der das biologische Gewebe umgreift und ein Gegenlager für den einzubringenden Blindniet bildet.

16. Blindniet nach einem der vorhergehenden Ansprüche, **dadurch gekennzeichnet, dass** die Spitze **(2)** des Trägers **1** aus einer resorbierbaren Magnesiumlegierung, vorzugsweise Magnesium AZ91 besteht.

## Claims

1. A self-piercing blind rivet for apposing biological tissue comprising a carrier (1) on which an axially fixed distal clip (5) and a proximal clip (6) being axially displaceable in the distal direction, wherein the carrier (1) has a coupling element (4) on the proximal end, into which an actuating element (7) can be hung up for displacing the clips relatively to each other, which can be unhooked from the coupling element for removing the rivet after its setting and wherein the distal end of the blind rivet is designed as a tip (2) in order to penetrate the tissue layers and wherein it is possible to hold the blind rivet in a guide tube (8) and can be pushed out of it for penetrating the tissue, **characterized in that** the carrier (1) is altogether formed as a tube and is formed as the tip (2) at its distal end so that several blind rivet can be magazined in such a way that the proximal end of the preceding blind rivet is insertable into the tip of the subsequent blind rivet.

2. The blind rivet according to claim 1, **characterized in that** the actuating element (7) is rope-shaped and has a locking element (7b) at its distal end which engages into the coupling element (4).

3. The blind rivet according to claim 1 or 2, **characterized in that** several rivets can be disposed one behind the other and are magazined in the guide tube (8) in its longitudinal direction, the proximal end of a preceding rivet being supported on the distal end of a subsequent rivet and that the actuating element (7) extends through the tube-shaped carriers (1, 9) of all magazined rivets and can be hooked into the coupling element (4) of the subsequent rivet after the unhooking of the foremost set rivet from the coupling element (4).

4. The blind rivet according to any of the preceding claims, **characterized in that** the blind rivet is inserted into the guide tube (8) in the instrument channel of an endoscope.

5. The blind rivet according to claim 4, **characterized in that** the guide tube (8) is the instrument channel of the endoscope.

6. The blind rivet according to claim 1, **characterized in that** locking teeth (3) are located on the tube-shaped carrier (1) which allows an axial relative movement of the proximal clip (6) only in the distal direction.

7. The blind rivet according to any of the preceding claims, **characterized in that** the coupling element (4) is pulled with the actuating element (7) for closing the blind rivet, whereby the proximal clip (6) is displaced in the distal direction.

8. The blind rivet according to any of the preceding claims, **characterized in that** the tip (2) is covered at the distal end of the blind rivet.

9. The blind rivet according to any of the preceding claims, **characterized in that** the carrier (1) comprises tapers and solid-state joints which are formed in such a way that the freedom of movement of the guide tube is not restricted.

10. The blind rivet according to any of the preceding claims, **characterized in that** biocompatible materials are used for the component of the rivet, at least the carrier (1) being bioresorbable so that the rivet maintains the closing force until the tissue itself is load-bearing.

11. The blind rivet according to claim 10, **characterized in that** the tip (2) at the distal end of the blind rivet is resorbed substantially more quickly than the remaining rivet in order to minimize the traumatization of the tissue or the surrounding organs.

12. The blind rivet according to claim 10, **characterized in that** the tip (2) on the distal end of the blind rivet is thrown off after the setting of the blind rivet in order to minimize the traumatization of the tissue or the surrounding organs.

13. The blind rivet according to claim 2, **characterized in that** the tip (2) at the distal end of the blind rivet is folded after the setting of the blind rivet in order to minimize the traumatization of the tissue or the surrounding organs.

14. The blind rivet according to any of the preceding claims, **characterized in that** the proximal clip (6) is combined with a medical instrument, e.g. a drug dispenser.

15. The blind rivet according to any of the preceding claims, **characterized in that** a holder-up is provided for fastening the blind rivet, which encompasses the biological tissue and forms a thrust bearing for the blind rivet to be introduced.

16. The blind rivet according to any of the preceding claims, **characterized in that** the tip (2) of the carrier (1) consists of a resorbable magnesium alloy, preferably magnesium AZ91.

## Revendications

1. Rivet aveugle autoperçant pour l'adaptation de tissu biologique, comportant un support (1) sur lequel sont agencées une agrafe distale (5) axialement fixe et une agrafe proximale (6) axialement déplaçable en direction distale, le support (1) présentant à l'extrémité proximale un élément d'accouplement (4) dans lequel un élément d'actionnement (7) peut être accroché pour déplacer les agrafes l'une par rapport à l'autre, et l'extrémité distale du rivet aveugle étant réalisée sous forme de pointe (2) pour pénétrer dans les couches de tissu, et le rivet aveugle pouvant être maintenu dans un tube de guidage (8) et extrait hors de celui-ci pour pénétrer dans le tissu, **caractérisé en ce que** le support (1) est réalisé dans l'ensemble en forme de tube et, à son extrémité distale, en tant que la pointe (2), de telle sorte que plusieurs rivets aveugles peut être emmagasinés de telle sorte que l'extrémité proximale d'un rivet aveugle précédent peut être enfichée dans la pointe d'un rivet aveugle suivant.

2. Rivet aveugle selon la revendication 1, **caractérisé en ce que** l'élément d'actionnement (7) est en forme de câble et présente à son extrémité distale un élément d'enclenchement (7b) s'engageant dans l'élément d'accouplement (4).

3. Rivet aveugle selon la revendication 1 ou 2, **caractérisé en ce que** plusieurs rivets peuvent être agencés les uns derrière les autres et emmagasinés dans le tube de guidage (8) dans sa direction longitudinale, l'extrémité proximale d'un rivet précédent prenant appui sur l'extrémité distale d'un rivet suivant, et **en ce que** l'élément d'actionnement (7) s'étend à travers les supports (1, 9) en forme de tube de tous les rivets emmagasinés et peut être accroché dans l'élément d'accouplement (4) du rivet suivant après avoir été décroché hors de l'élément d'accouplement (4) du rivet placé en premier.

4. Rivet aveugle selon l'une quelconque des revendications précédentes, **caractérisé en ce que** le rivet aveugle est introduit dans le tube de guidage (8) dans le canal instrumental d'un endoscope.

5. Rivet aveugle selon la revendication 4, **caractérisé en ce que** le tube de guidage (8) est le canal instrumental d'un endoscope.

6. Rivet aveugle selon la revendication 1, **caractérisé en ce que** le support (1) en forme de tube se trouve dans une denture d'enclenchement (3) qui permet un mouvement relatif axial de l'agrafe proximale (6) seulement en direction distale.

7. Rivet aveugle selon l'une quelconque des revendications précédentes, **caractérisé en ce que** pour fermer le rivet aveugle avec l'élément d'actionnement (7), on tire sur l'élément d'accouplement (4), ce qui déplace l'agrafe proximale (6) en direction distale.

8. Rivet aveugle selon l'une quelconque des revendications précédentes, **caractérisé en ce que** la pointe (2) est recouverte à l'extrémité distale du rivet aveugle.

9. Rivet aveugle selon l'une quelconque des revendications précédentes, **caractérisé en ce que** le support (1) présente des rétrécissements et des articulations à corps solide qui sont réalisés de telle sorte que la liberté de mouvement du tube de guidage n'est pas restreinte.

10. Rivet aveugle selon l'une quelconque des revendications précédentes, **caractérisé en ce que** pour les composants du rivet sont utilisés des matériaux biocompatibles, le support (1) étant au moins biorésorbable, de telle sorte que le rivet maintient la force de fermeture aussi longtemps que le tissu est résistant.

11. Rivet aveugle selon la revendication 10, **caractérisé en ce que** la pointe (2) à l'extrémité distale du rivet aveugle est sensiblement plus rapidement résorbable que le reste du rivet, pour minimiser le traumatisme du tissu ou des organes environnants.

12. Rivet aveugle selon la revendication 10, **caractérisé en ce que** la pointe (2) à l'extrémité distale du rivet aveugle est rejetée après avoir placé le rivet aveugle, pour minimiser le traumatisme du tissu ou des organes environnants.

13. Rivet aveugle selon la revendication 2, **caractérisé en ce que** la pointe (2) à l'extrémité distale du rivet aveugle est rabattue après avoir placé le rivet aveugle, pour minimiser le traumatisme du tissu ou des organes environnants.

14. Rivet aveugle selon l'une quelconque des revendications précédentes, **caractérisé en ce que** l'agrafe proximale (6) est combinée avec un appareil médical, par exemple un distributeur de médicaments.

15. Rivet aveugle selon l'une quelconque des revendications précédentes, **caractérisé en ce que** pour fixer le rivet aveugle est prévu une contre-bouterolle qui entoure le tissu biologique et qui forme un palier-support pour le rivet aveugle à monter.

16. Rivet aveugle selon l'une quelconque des revendications précédentes, **caractérisé en ce que** la pointe (2) du support (1) est en un alliage de magnésium résorbable, de préférence en magnésium AZ 91.
